# EUROPÄISCHE PATENTSCHRIFT

(11) **EP 0 659 725 B1**
(45) Veröffentlichungstag und Bekanntmachung des Hinweises auf die Patenterteilung: **02.05.1997**
(21) Anmeldenummer: 94119898.8
(22) Anmeldetag: 16.12.1994
(51) Int. Cl.: C07C 45/50, C07C 5/25, C07C 47/02, C07C 11/02

(54) **Verfahren zur Herstellung von in alpha-Stellung durch einen Alkylrest substituierten Aldehyden**
Process for the preparation of aldehydes substituted by an alkylradical in alpha position
Procédé pour la préparation d'aldehydes substitués par un radical alkyl en position alpha

(30) Priorität: 23.12.1993 DE 4344064
(43) Veröffentlichungstag der Anmeldung: 28.06.1995
(73) Patentinhaber: HOECHST AKTIENGESELLSCHAFT, 65926 Frankfurt am Main (DE)
(72) Erfinder: Springer, Helmut, D-46145 Oberhausen (DE); Weber, Jürgen, Dr., D-46147 Oberhausen (DE)

(56) Entgegenhaltungen:
- WO-A-84/03697
- FR-A- 2 395 245
- NL-A- 7 105 267
- US-A- 4 178 313
- US-A- 4 587 374

## Beschreibung

Die Erfindung betrifft ein Verfahren zur Herstellung von in α-Stellung durch einen Alkylrest substituierten Aldehyden mit 8 bis 17 Kohlenstoffatomen im Molekül durch Hydroformylierung der entsprechenden, um ein Kohlenstoffatom ärmeren, Olefine.

Aldehyde mit 8 bis 17 Kohlenstoffatomen im Molekül, die in α-Stellung zur Carbonylgruppe durch Alkylreste substituiert sind, haben als Zwischenprodukte technische Bedeutung. Aus ihnen werden zum Beispiel durch Hydrierung die entsprechenden Alkohole, durch Oxidation Carbonsäuren und durch aminierende Hydrierung Amine hergestellt. Die genannten Verbindungsklassen finden als Rohstoffe zum Beispiel zur Herstellung von Additiven für Schmiermittel oder Kunststoffe Anwendung.

Die Herstellung von Aldehyden durch Hydroformylierung olefinisch ungesättigter Verbindungen ist eine bekannte und industriell im großem Maßstab und in verschiedenen Varianten ausgeübte Reaktion. Üblicherweise geht man von endständigen Olefinen aus, die in großen Mengen als Raffinerieprodukte zur Verfügung stehen und die überwiegend in endständige Aldehyde überführt werden. Für spezielle Anwendungen benötigt man jedoch auch in α-Stellung zur Carbonylgruppe verzweigte Aldehyde, die durch Hydroformylierung aus Olefinen mit innenständiger Doppelbindung gewonnen werden können.

Bei der Verarbeitung von Rohölen fallen Olefine mit innenständiger Doppelbindung nur in begrenzten Mengen an. Sie lassen sich jedoch aus Olefinen, die eine Doppelbindung am Ende der Kohlenstoffkette enthalten, durch Isomerisierung herstellen. Diese Reaktion wird durch verschiedene Katalysatoren beeinflußt. Bekannt ist zum Beispiel die isomerisierende Wirkung von Carbonylen der Metalle Eisen, Kobalt und Nickel über die Asinger und Berg in Chem. Ber. 88,445 (1955) berichten. Über ähnliche Ergebnisse, gefunden mit Eisencarbonylen als Katalysatoren referiert Manuel in JOC. 27,3941 (1962). Schließlich beschreiben Asinger, Fell und Collin in Chem. Ber. 96,716 (1963) die Doppelbindungsisomerisierung unter dem Einfluß von sauren, basischen und neutralen Verbindungen.

Die modernen Hydroformylierungsverfahren arbeiten mit Rhodium oder Rhodiumverbindungen als Katalysatoren, die in sehr geringer Konzentration, d. h. in einer Menge von etwa 1 bis etwa 20 Gew.-ppm bezogen auf das Olefin, im Reaktionsgemisch vorliegen. Um einer Beeinträchtigung der Aktivität des Katalysators durch Schadstoffe zu begegnen, setzt man daher sehr reine Ausgangsmaterialien, Synthesegas und Olefin, ein. Die Reinigung der Reaktanten ist unter diesen Umständen für moderne Oxosynthesen ein kostenbestimmender Faktor. Durch verfahrenstechnische Maßnahmen oder Abwandlung des Reaktionsablaufs ist man daher bemüht, aufwendige Reinigungsschritte zu vermeiden, ohne die Ausbeute oder die Qualität des Reaktionsproduktes zu mindern. Auf die Herstellung in α-Stellung verzweigter Aldehyde bezogen bedeutet diese Forderung, daß durch Isomerisierung gewonnene innenständige Olefine möglichst ohne aufwendige zusätzliche Maßnahmen in der Oxosynthese eingesetzt werden sollen.

Es bestand daher die Aufgabe, die Isomerisierung entständiger Olefine zu innenständigen Olefinen und deren formylierung zu α-alkylverzweigten Aldehyden zu einem samtprozeß zu kombinieren, der technisch einfach durchzuführen ist, selektiv arbeitet und die gewünschten Produkte in hoher Ausbeute ergibt.

Die Erfindung besteht in einem Verfahren zur Herstellung von in α-Stellung durch einen Alkylrest substituierten Aldehyden mit 8 bis 17 Kohlenstoffatomen durch Hydroformylierung von Olefinen mit 7 bis 16 Kohlenstoffatomen mit innenständiger Doppelbindung. Es ist dadurch gekennzeichnet, daß Olefine mit vorwiegend endständiger Doppelbindung bei 160 bis 210°C in flüssiger Phase in Gegenwart von Eisencarbonyl isomerisiert und ohne Abtrennung des Isomerisierungskatalysators bei 80 bis 200°C und einem Druck von 5 bis 50 MPa in Gegenwart von Rhodium als Katalysator hydroformyliert werden.

Der besondere Vorteil des erfindungsgemäßen Verfahrens besteht in der unmittelbaren Hydroformylierung des merisierungsgemisches, ohne vorherige Reinigung des produktes und ohne Abtrennung des Isomerisierungskatalysators. Diese Reaktionsführung überrascht in mehrfacher Hinsicht. So sind Eisencarbonyle als sehr wirksame Aldolisierungskatalysatoren bekannt mit der Folge, daß man bemüht ist, eine Verunreinigung des Hydroformylierungsproduktes mit diesen Eisenverbindungen zu vermeiden um die Aldehydausbeute durch Bildung von Aldolen nicht zu beeinträchtigen. Überdies sind Eisencarbonyl auch Hydroformylierungskatalysatoren, die ähnlich Kobalt die Bildung geradkettiger Aldehyde fördern, also unter lierungsbedingungen der Isomerisierung endständiger zu innenständigen Olefinen entgegenwirkt. Diese Tendenz sollte durch Verwendung von Rhodium als Hydroformylierungskatalysator verstärkt werden, weil auch unter dem Einfluß dieses Metalls endständige Doppelbindungen schneller hydroformyliert werden als innenständige. Es ist daher zu erwarten, daß das Gleichgewicht zwischen end- und innenständigen Olefinen laufend zu gunsten der endständigen Olefine verschoben wird und geradkettige Aldehyde im Endprodukt dominieren. Tatsächlich werden aber nach dem neuen Verfahren in der Hauptsache verzweigte Aldehyde gebildet.

Im ersten Verfahrensschritt werden α-Olefine mit 7 bis 16 Kohlenstoffatomen im Molekül oder Gemische die solche Olefine enthalten isomerisiert. Als Einsatzmaterial verwendet man die z. B. durch Cracken von Wachs oder durch Ethylen-Oligomerisierung erhaltenen ungesättigten Kohlenwasserstoffe in handelsüblicher Form. Eine vorherige Reinigung ist nicht erforderlich. Je nach dem gewünschten Produkt setzt man Olefine einheitlicher Molekülgröße oder Olefingemische ein.

Die Isomerisierung erfolgt in flüssiger Phase in Gegenwart von Eisencarbonyl, das in einer Konzentration von 50 bis 1000 Gew.-ppm, vorzugsweise 200 bis 500 Gew.-ppm bezogen auf Olefin vorliegt. Als Eisencarbonyl werden die unter den jeweiligen Reaktionsbedingungen beständigen Carbonylverbindungen verstanden wie Eisenpentacarbonyl oder Trieisendodecacarbonyl. Die Reaktion läuft bei Temperaturen von 160 bis 210°C vorzugsweise 170 bis 190 °C in flüssiger Phase ab. Je nach der angewandten Temperatur und dem eingesetzten Olefin arbeitet man daher drucklos oder unter Druck. Üblicherweise erfordert die Isomerisierung eine Reaktionszeit von 10 bis 180 insbesondere 30 bis 60 Minuten. Das Olefin kann in einem inerten Lösungsmittel, z. B. in einem aliphatischen Kohlenwasserstoff wie Cyclohexan oder einem aromatischen Kohlenwasserstoff wie Toluol gelöst sein, jedoch ist im allgemeinen die Anwesenheit eines Lösungsmittels nicht erforderlich.

Das Isomerisierungsgemisch wird erfindungsgemäß unmittelbar hydroformyliert. Die Umsetzung des ungesättigten Kohlenwasserstoffs mit Wasserstoff und Kohlenmonoxid erfolgt bei Temperaturen von 80 bis 200°C und Drücken von 5 bis 50 MPa in Gegenwart von 1 bis 20 Gew.-ppm Rhodium bezogen auf das Olefin.

Besonders günstige Ergebnisse werden erzielt bei Reaktionstemperaturen von 120 bis 150°C und Drücken von 20 bis 30 MPa und Rhodium-Konzentrationen von 2 bis 5 Gew.-ppm bezogen auf das Olefin.

Das als Katalysator verwendete Rhodium wird in das Reaktionsgemisch als Metall oder in Form von anorganischen oder organischen Verbindungen, z. B. als Rhodiumoxid, Rhodiumtrichlorid, als Nitrat, Sulfat oder als Ethylhexanoat zugegeben. Unter den Reaktionsbedingungen bilden sich lösliche, katalytisch aktive Rhodiumcarbonylverbindungen, die man natürlich auch vor der Hydroformylierng herstellen und in dieser Form dem Reaktionsgemisch zuführen kann. Kohlenmonoxid und Wasserstoff werden in den bei der Hydroformylierung üblichen Mengenverhältnissen angewandt.

Die Hydroformylierung der Olefine kann in Gegenwart eines inerten Lösungsmittels durchgeführt werden, doch ist seine Gegenwart im allgemeinen nicht erforderlich. Geeignete Lösungsmittel sind aliphatische und aromatische Kohlenwasserstoffe wie Benzol, Toluol, Cyclohexan und cyclische Ether z. B. Tetrahydrofuran. Zweckmäßig verwendet man für Isomierisierung und Hydroformylierung dasselbe Lösungsmittel sofern in beiden Reaktionsstufen ein Lösungsmittel eingesetzt wird.

Die Abtrennung des Rhodium- und des Eisen-Katalysators aus dem aldehydischen Reaktionsprodukt erfolgt nach bekannten Verfahren. So zersetzen sich die Katalysatoren zumindest teilweise bereits beim Entspannen des Druckreaktors und können einfach abfiltriert werden. Eine andere Methode zu ihrer Entfernung besteht in einer Behandlung des Hydroformylierungsproduktes mit Wasser oder Wasserdampf bei erhöhten Temperaturen, wobei zweckmäßig 5 bis 50 Vol.-% Wasser oder die entsprechende Menge Wasserdampf, bezogen auf die eingesetzte Reaktionsmischung angewandt und Temperaturen zwischen 80 und 200°C gewählt werden. Dabei fallen die Metalle, üblicherweise im Gemisch mit ihren Oxiden, in der wässrigen Phase aus und können abfiltriert werden. Rhodium und Rhodiumverbindungen lassen sich von Eisen und Eisenverbindungen z. B. durch Behandlung des Filterrückstandes mit einer Mineralsäure trennen. Der rhodiumhaltige Rückstand kann ohne spezielle Reinigungsmaßnahmen erneut in der Hydroformylierungsstufe eingesetzt werden. Nach destillativer Abtrennung des Hydroformylierungsproduktes aus dem Reaktionsgemisch, z. B. durch Flash-Verdampfung, können Reste des rhodiumhaltigen Materials aus dem Destillationsrückstand wiedergewonnen werden.

Die erfindungsgemäß gewonnenen Aldehyde können als solche verwendet oder z. B. zu Alkoholen hydriert, zu Carbonsäuren oxidiert oder zu Aminen aminierend hydriert werden.

Durch die nachfolgenden Beispiele wird das erfindungsgemäße Verfahren näher erläutert.

### Beispiel 1: Herstellung isomerer Tridecanale aus 1-Dodecen

2.800 g eines Dodecengemisches mit einem Gehalt von 96,75 Gew.-% 1-Dodecen werden in Gegenwart von 1,4 g (500 Gew.-ppm) Eisenpentacarbonyl auf 170°C erhitzt. Die Reaktion wird nach 60 min abgebrochen. Man erhält ein Gemisch isomerer Dodecene folgender Zusammensetzung (in Gew.-%):

| | |
|---|---|
| 1-Dodecen | 2,1 |
| 2-Dodecen | 28,8 |
| 3-Dodecen | 20,0 |
| 4-,5-,6-Dodecen | 46,5 |
| Sonstige | 2,6 |

700 g des Isomerisierungsgemisches werden ohne Abtrennung des Isomerisierungskatalysators und nach Zusatz von 3,5 mg (5 Gew.-ppm) Rhodium in Form des 2-Ethylhexanoats in einem Autoklaven mit einem äquimolaren Kohlenmonoxid-/Wasserstoffgemisch unter einem Druck von 26 bis 27 MPa auf 130°C erhitzt. Die Reaktion wird nach Beendigung der Gasaufnahme (d. h. nach etwa 1,5 h Reaktionszeit) abgebrochen. Das Reaktionsprodukt wird abgekühlt, entspannt, nach Abtrennen des Isomerisierungs- und des Hydroformylierungskatalysators destilliert und anschließend analysiert.

Bei einem Umsatz von 99,9% erhält man ein Gemisch isomerer Tridecanale, das wie folgt zusammengesetzt ist (in Gew.-%):

| | |
|---|---|
| 2-Pentyloctanal | 34,9 |
| 2-Butylnonanal | |
| 2-Propyldecanal | 15,3 |
| 2-Ethylundecanal | 16,4 |
| 2-Methyldodecanal | 23,3 |
| n-Tridecanal | 7,8 |
| Dodecen | 0,1 |
| Sonstige | 2,2 |

### Beispiel 2: Herstellung isomerer Heptadecanale aus 1-Hexadecen

2116 g eines Hexadecengemisches mit einem Gehalt von 92,5 Gew.-% 1-Hexadecen werden in Gegenwart von 1,06 g (500 Gew.-ppm) Eisenpentacarbonyl auf 180°C erhitzt. Die Reaktion wird nach 120 Min. abgebrochen. Man erhält ein Gemisch isomerer Hexadecene folgender Zusammensetzung (in Gew.-%):

| | |
|---|---|
| 1-Hexadecen | 5,0 |
| 2-Hexadecen | 15,1 |
| 3-Hexadecen | 12,1 |
| 4-,5-,6-,7-,8-Hexadecen | 60,3 |
| Sonstige | 7,5 |

1480 g des Isomerisierungsgemisches werden ohne Abtrennung des Isomerisierungskatalysators und nach Zusatz von 7,4 mg (5 Gew.-ppm) Rhodium in Form des 2-Ethylhexanoats in einem Autoklaven mit einem äquimolaren Kohlenmonoxid/Wasserstoffgemisch unter einem Druck von 26 bis 27 MPa auf 130°C erhitzt. Die Reaktion wird nach Beendigung der Gasaufnahme (d.h. nach etwa 1 h Reaktionszeit) abgebrochen. Das Reaktionsprodukt wird abgekühlt, entspannt, nach Abtrennen des Isomerisierungs- und des Hydroformylierungskatalysators destilliert und anschließend analysiert. Bei einem Umsatz von 99,5 % erhält man ein Gemisch isomerer Heptadecanale, das sich wie folgt zusammensetzt (in Gew.-%):

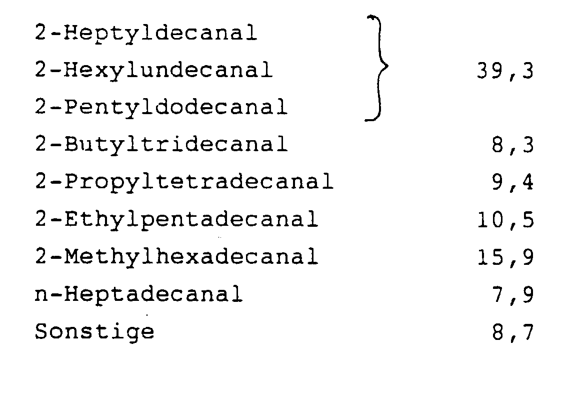

## Patentansprüche

1. Verfahren zur Herstellung von in α-Stellung durch einen Alkylrest substituierten Aldehyden mit 8 bis 17 Kohlenstoffatomen durch Hydroformylierung von Olefinen mit 7 bis 16 Kohlenstoffatomen mit innenständiger Doppelbindung dadurch gekennzeichnet, daß Olefine mit vorwiegend endständiger Doppelbindung bei 160 bis 210°C in flüssiger Phase in Gegenwart von Eisencarbonyl isomerisiert und ohne Abtrennung des Isomerisierungskatalysators bei 80 bis 200°C und einem Druck von 5 bis 50 MPa in Gegenwart von Rhodium als Katalysator hydroformyliert werden.

2. Verfahren nach Anspruch 1 dadurch gekennzeichnet, daß die Isomerisierung in Gegenwart von 50 bis 1000 Gew.-ppm, vorzugsweise 200 bis 500 Gew.-ppm Eisencarbonyl bezogen auf Olefin erfolgt.

3. Verfahren nach Anspruch 1 oder 2 dadurch gekennzeichnet, daß die Isomerisierung bei 170 bis 190°C erfolgt.

4. Verfahren nach einem oder mehreren der Ansprüche 1 bis 3 dadurch gekennzeichnet, daß die Hydroformylierung in Gegenwart von 1 bis 20 Gew.-ppm, vorzugsweise 2 bis 5 Gew.-ppm Rhodium bezogen auf Olefin erfolgt.

5. Verfahren nach einem oder mehreren der Patentansprüche 1 bis 4 dadurch gekennzeichnet, daß die Hydroformylierung bei Temperaturen von 120 bis 150°C erfolgt.

6. Verfahren nach einem oder mehreren der Patentansprüche 1 bis 5 dadurch gekennzeichnet, daß die Hydroformylierung bei Drücken von 20 bis 30 MPa erfolgt.

## Claims

1. A process for preparing aldehydes having from 8 to 17 carbon atoms and substituted in the α position by an alkyl radical by means of hydroformylation of olefins having from 7 to 16 carbon atoms and an internal double bond, which comprises isomerizing olefins having a predominantly terminal double bond at from 160 to 210°C in the liquid phase in the presence of iron carbonyl and, without separating off the isomerization catalyst, hydroformylating the isomerization mixture at from 80 to 200°C and a pressure of from 5 to 50 MPa in the presence of rhodium as catalyst.

2. The process is claimed in claim 1, wherein the isomerization is carried out in the presence of from 50 to 1000 ppm by weight, preferably from 200 to 500 ppm by weight, of iron carbonyl, based on olefin.

3. The process as claimed in claim 1 or 2, wherein the isomerization is carried out at from 170 to 190°C.

4. The process as claimed in one or more of claims 1 to 3, wherein the hydroformylation is carried out in the presence of from 1 to 20 ppm by weight, preferably from 2 to 5 ppm by weight, of rhodium, based on olefin.

5. The process as claimed in one or more of claims 1 to 4, wherein the hydroformylation is carried out at temperatures of from 120 to 150°C.

6. The process as claimed in one or more of claims 1 to 5, wherein the hydroformylation is carried out at pressures of from 20 to 30 MPa.

## Revendications

1. Procédé de préparation d'aldéhydes contenant 8 à 17 atomes de carbone et substitués en position a par un groupe alkyle par hydroformylation d'oléfines contenant 7 à 16 atomes de carbone et à double liaison interne, caractérisé en ce que l'on isomérise des oléfines à double liaison principalement terminale à des températures de 160 à 210°C en phase liquide en présence d'un fer-carbonyle et, sans séparer le catalyseur d'isomérisation, on soumet à hydroformylation à des températures de 80 à 200°C sous une pression de 5 à 50 MPa en présence de rhodium qui sert de catalyseur.

2. Procédé selon la revendication 1, caractérisé en ce que l'isomérisation est réalisée en présence de 50 à 1 000 ppm en poids, de préférence 200 à 500 ppm en poids, de fer-carbonyle par rapport à l'oléfine.

3. Procédé selon la revendication 1 ou 2, caractérisé en ce que l'isomérisation est réalisée à des températures de 170 à 190°C.

4. Procédé selon l'une ou plusieurs des revendications 1 à 3, caractérisé en ce que l'hydroformylation est réalisée en présence de 1 à 20 ppm en poids, de préférence de 2 à 5 ppm en poids de rhodium par rapport à l'oléfine.

5. Procédé selon l'une ou plusieurs des revendications 1 à 4, caractérisé en ce que l'hydroformylation est réalisée à des températures de 120 à 150°C.

6. Procédé selon l'une ou plusieurs des revendications 1 à 5, caractérisé en ce que l'hydroformylation est réalisée sous des pressions de 20 à 30 MPa.
